# EUROPEAN PATENT SPECIFICATION

(11) **EP 2 198 035 B1**
(45) Date of publication and mention of the grant of the patent: **11.04.2012**
(21) Application number: 07785503.9
(22) Date of filing: 30.07.2007
(51) Int. Cl.: C12P 7/10, C12P 17/04, C12P 19/02, C13K 1/02, C07G 1/00, D21C 3/04, D21C 3/24, D21C 11/00

(54) **Method and equipment for production of glucose, ethanol, furfural, furane and lignin from renewable raw materials**
Verfahren und Einrichtung zur Herstellung von Glucose, Ethanol, Furfural, Furan und Lignin aus erneuerbaren Rohstoffen
Procédé et équipement de production de glucose, d'éthanol, de furfural, de furane et de lignine à partir de matières premières renouvelables

(43) Date of publication of application: 23.06.2010
(73) Proprietor: Kmps Financial Group, S.R.O., 170 00 Praha 7 (CZ)
(72) Inventor: KRATOCHVÍL, Zdenek, 160 00 Praha 6 (CZ); RYCHTÁR, Libor, 708 00 Ostrava-Poruba (CZ); MACHEK, Frantisek, 150 00 Praha 5 (CZ); BOUSKA, Frantisek, 150 00 Praha 5 (CZ)
(74) Representative: Smrckova, Marie
(86) International application number: PCT/CZ2007/000076
(87) International publication number: WO 2009/015614

(56) References cited:
- EP-A- 1 130 085
- WO-A-96/25553
- WO-A-2006/024242
- CZ-B6- 300 865
- GB-A- 570 903
- GB-A- 660 773
- GB-A- 903 615
- KARIMI ET AL: "Conversion of rice straw to sugars by dilute-acid hydrolysis" BIOMASS AND BIOENERGY, PERGAMON, OXFORD, GB, vol. 30, no. 3, March 2006 (2006-03), pages 247-253, XP005294029 ISSN: 0961-9534
- LEE K C ET AL: "Hybrid process for the conversion of lignocellulosic materials.", APPLIED BIOCHEMISTRY AND BIOTECHNOLOGY APR 1997 LNKD- PUBMED:9204515, vol. 66, no. 1, April 1997 (1997-04), pages 1-23, ISSN: 0273-2289

## Description

### Field of the Invention

Invention is solving the complex method of working up of ligno-cellulosic and amylaceous materials onto the monosaccharides, glucose, fuel alcohol, furfural, furane, acetic-acid, formic-acid and lignin. According to the origin and composition of ligno-cellulosic materials, for example the proportion of hemicellulose and cellulose itself can be possible to prepare the conditions of thermo-compressive hydrolysis as optimum. The created monosaccharides are the basic energetic raw material for the fermenting preparation of ethanol, lactic-acid and next fermented products.

### Background of the Invention

The lack of fossil raw material sources is becoming a potential obstacle which can break economical and social development of most countries. Today's systems of organic chemical productions are mostly based on fossil raw materials and non-biologic technologies. Fossil raw materials, namely oil and earth gas are step by step being exhausted.

Beside the fossil sources of raw materials there is the renewable organic mass for disposal nowadays and namely in the future. Organic (biologic) mass further LCM (ligno--cellulosic materials) is the mostly used renewable source of energy now, and it has huge part in all over the world production. From the quality point of view there is the situation of LCM usage completely unsatisfactory. Namely in the economically less developed countries is the wood main material for heating, and the way of wood combustion is uneconomical. The usage of LCM materials from the agricultural production is not on demanded and possible level.

Main producers of LCM materials are the prior sources of agricultural production (various kinds of straw), and the waste from wood and forest industry. If these sources shall be divided into the use in three stages, their usage will be as follows:
- in first stage the sources from agricultural production shall be used
- in second stage the sources from wood industry shall be used
- in third stage the waste materials from forests shall be used

The mostly advanced stage is the verification of LCM materials from primary agricultural production, by which there were reached very good technologic and economic results.

Several following paragraphs are describing certain hydrolytic and dehydratation methods:
The company Quaker Oats used the method of discontinous hydrolysis of ligno-celluloses with sulphur-acid (5% water solution) for production of furaldehyd, with the help of temperature 145 till 170 °C.

In the firm Agrifuran there is using the water extract of superphosphate containing 45 wt.% P₂O₅, which is added directly into autoclave.

Sweden company Defibrator designed the continual hydrolysis. They used single-stage expansion, and the raw material is impregnated by sulphur-acid before the hydrolysis.

There is known a CZ patent No. 191945 which is solving the problem with the help of continual two-stage hydrolysis, and the added sulphur-acid. At first stage the hydrolysis is carried out in temperature 150 till 200 °C with concentration of sulphur-acid higher than 10 wt.%. At second stage the same temperature is used, and the concentration of sulphur-acid is recommended up to 5 wt.%.

All above mentioned methods of fural production have the common imperfection. It is comparatively small amount of final product, maximum 30 till 45%, and insufficient upvaluation some parts of used raw materials, namely of rest phase of ligno-cellulosis.

When the fluidic method of furaldehyd production is used, the lowering of final product amount is caused by thermo-oxidative decomposition of furaldehyd in the reaction with air oxygen.

A Swiss patent No. CH 678183 A5 has introduced the acidic hydrolysis of raw materials which contain the pentosans, with the help of sulphur-acid in concentration 2 wt.%, and temperature of 170 till 230°C.

The pentose fraction is utilized all over the world for fural production. Older attempts leading to fural production were based on the pressure and heat applied onto the raw material. Original patents La Forge used the dehydratic reactions with the help of certain organic acid, which was released from the vegetable raw material by the action of superheated vapour (CH₃COOH, HCOOH). The company Quaker Oats started to use sulphur-acid (5% water solution), and the temperature 145 till 147 °C.

Beside this technology there is several continuous technologies, which can be divided as follows:
a) direct (single-stage)
b) indirect (two-stage and more-stage).

Indirect methods are two-stage methods, in first stage the solution of saccharoses (with the help of prehydrolysis, hydrolysis, delignification or extraction) has been prepared, and this solution is working up in next stage with the help of dehydratation reaction. There are methods by which the fural in first step from the relief gases can be separating, but most amount of fural is created in second stage of prehydrolysis dehydratation.

During above mentioned production methods of furfural is very hard to prevent the reactions of furfural with other parts creating from this process, and other degradation processes. The creation of furfural and its drawing off from the reaction zone is determined by its diffusion from vegetable materials, in which is creating. There is not possible to remove the air oxygen from the reaction space, what is the main disadvantage of direct methods. Air oxygen presence can cause as much as 10% lowering of fural production.

According to the method of vegetable raw material usage, these methods can be categorized:
- fural and hexosis hydrolysate (this variant needs the highest demands for process technology from the point of view of reaction kinetics, temperature and pressure, and so on)
- fural and cellulose (fibrous materials)
- fural and binding agent, carbon (activated carbon)
- fural and fertilizer

Next, there are announced some of used fural production methods (eventually together with other products):
Company Quaker Oats is using the discontinual charging of ligno-cellulosis materials into the autoclave, where 5% sulphur-acid is feeding as well, and the lowest hydromodule is kept, it means as much as 0.5. Rest from hydrolysis is drying at dryer, and it is used as solid fuel or fertilizer. The neutralization of this rest is carrying out by the ammonia.

The firm Thermodynamik has developed similar method of fural production as Quaker Oats, which uses the wood from leaf trees. Water extract of superphosphate (containing 45% of P₂O₅) as additive into autoclave, can be used instead of sulphur-acid (method of Agrifuran).

According to the company Defibrator, the wood chips shall be exposed by the high pressure vapour in continual pressure equipment. The raw material shall be drawn up from the autoclave to the expander, where the pressure shall be lowered to normal athmospheric pressure. There shall be separated fural and water vapour from solid rest which shall be taken away from the expander by the help of endless screw.

Common imperfection of above mentioned methods is the shortage of process efficiency which cannot reach 45% of theoretic calculation, and insufficient valorization of other parts (namely cellulose). In the fluid layer there occurs the degradation decomposition of fural when it is contacting with oxygen. The other difficult reaction is creation of glycosans.

Causes of the breaking down of 2-furaldahyd (furfural) production all over the world can be characterized on the base of reasonable informations as follows:
- breaking down of machinery (not very often cases, for example the continual feeder was broken sometimes)
- breaking down of technology (it is resulted in very complicated mechanism of 2-furaldehyd creation and decomposition with many following reactions, which are complicated by diffusion reactions in entering raw material, and the specific hydrodynamics of water vapour in the reaction equipment)
- low (or zero) profit of production resulting mainly in the usage of unsuitable technology for the given ligno-cellulosic material, its production capacity and the energetic demands.

There shall be necessary to develop or to invent cheaper and more effective methods of raw material conversion to the ecologic gas and fluid fuels at near future, which shall make -possible wider usage of biomass and renewable sources in whole, i.e. without limitation of the raw material source distance, and also higher flexibility for methods of their applications and without seasons vicissitudes.

As mentioned above, the methods for furaldehyd (fural) production, further the lignin and hydrolytic saccharides production. There has been concentrated attention to ethanol production based on the amylaceous and ligno-cellulosic materials.

In spite of the fact that nowadays'practice of usage of the fosile raw material for ethanol production is more effective, the greatest petrochemistry companies found their research and development workshops for investigation of new techologies based on renewable sources. The conference European on Bioethanol was engaged in the causes of the unfavourable effectivity of production methods, from the technologic and legal point of view. The sale price of bio-ethanol, which is being produced or will be produced from the corn or grain starch, is nearly the same as the price of purchasing raw materials. These materials are comparatively expensive but their advantage is in the easier techologically managed hydrolysis.

All above mentioned features has announced Mr. Philip W. Madson in his lecture "Bioethanol experiences in the USA" held at Lissa (Netherland) on May 1990. Finally he stated that the bio-ethanol production is on the limit of rentability, even when the technical progress continues in its development. His opinion is that new methods could solve the problem of production effectivity, namely when the government would support the legal provisions. During next several years there were issued several patents, but no one of them can solve the problem as a complex, and more economically.

The known method of bio-ethanol production has been described recently in US patent No. 4.564.595, which has been based on the acidic hydrolysis of predelignificated cellulose, and the following fermentation of created monosaccharides (namely glucose). Most of patents describes the ethanol fermentation with lower pressure, by which the separation of ethanol from the fermentation part is going on. The separation of ethanol can be accelerated by the bubling through carbon oxide. A disadvantage of this method may be the necessary delignification of ligno-cellulosic material, and low concentration of fermentable saccharides.

There is known European patent No. 0 101 190 named "Process for producing ethanol" of two authors Mr. Assarson and Mr. Nagasuy, who use the acidic parcial hydrolysis of starch for glucose production, and the glucose is then fermented, so that the ethanol is created. As the entering raw material they consider carbo-hydratic material adjusted by various methods (chemically modificated, derived, unmodificated and/or their mixtures). The cellulose theoretically belongs among some of these groups even when the authors do not mention it on their list of raw materials. But designed conditions of hydrolysis, mainly the temperature, cut out the cellulose from the list of usable raw materials. There is possible that during the temperature 167 °C, the hydrolysis of pentosan part can occure only, the ligno-cellulosic complex keeps intacted. This is the reason why only amylaceous raw material is stated on the list.

Japanese patent No. 59048090 named "Preparation of fuel alcohol" is trying to remove the high energetic demands of known methods. It is based on the fact that the monosaccharides can be prepared from renewable raw materials with the help of fermentation, and they are fermented to ethanol in the next step. The amylaceous materials are cracked with the help of the fibre fungi of genus *Aspergilus,* ligno-cellulosic materials like woods are treated with the help of distillery yeast, straw and similar materials with the help of *Bacillus natto.* All components shall be mixed in the rate 5:3:2, and the mixture shall be submitted to the alcohol fermentation. The evident disadvantage of this method is the fact that high-molecular saccharides must be submitted to the prefermentation cracking. This method needs next three fermentation units, and this type of fermentation goes on very slowly.

Designed technologies are based on the usage of amylaceous materials (namely corn and grain) untill nowadays.

Comparison and knowledges from conventional method of ethanol production based on renewable materials (grain, corn) supports the development, and caused that the new industrial branch was established. Nowadays, nearly 9% of petrol consumption in USA can be supplied as mixture containing 10% of ethanol.

Description concerning the production expenses of individual factory for ethanol production as fuel alcohol, which is working up the grain or corn by dry mills, are nearly the same as the sale price of ethanol product. If there are compared the profit and losses of production expenses including the equipment, working powers, energy etc., the loss shall be resulted.

The future projects of ethanol production shall be able to bring better evaluation of the adjacent products, not only ethanol itself, and profitable sale of ethanol as fuel alcohol. Separation and treatment of the adjacent products with low expenses, which are contained in non-amylaceous fraction of raw material (grain, corn), is the subject of great interest.

The second strategy which is subjected to the reasonable interest nowadays, is to use the entering raw materials with lower expenses, as for example cellulose, so that to reduce total expenses of raw material, and finally the expenses of fuel alcohol production.

There were many factories in USA using the boiling systems designed for the prior working up the starch, without any regard to critical demands for bacterial control. Typical grains can contain as much as 10 milion bacterial and mildew cells in one gram of raw material. This biological problem highly exceeds the limit for efficient fermentation. Many of these factories meet uncontrolable infections which caused the lowering of profit.

Procedure for the production of ethanol from lignocellulosic biomass shows EP 1 130 085 A1. It includes the states of the grinding the lignocellulosic biomass to a size of 15 - 30 mm, subjecting the product obtained to steam explosion pre-treatment at a temperature of 190 - 230 °C for between 1 and 10 minutes in a reactor, collecting the pre-treated material in a cyclone, and separating the liquid and solid fractions by filtration In a filter press, introducing the solid fraction in a fermentation deposit, adding a cellulose at a concentration of 15 UFP per gram of cellulose and 12.6 international Units β-glucosidase enzyme dissolved In citrate buffer bph 4.8, inoculating the fermentation deposit with a culture of the heat-tolerant bacteria *Kluyveromyces marxianus CECT 10875,* obtained by chemical mutagenesis from strain *DER* -26 of *Kluyveromyces marxianus* and shaking the mixture for 72 hours at 42 °C. Collection of the pre-treated material is provided in a cyclone.

In Biomass and Bioenergy ( Pergamon, Oxford GB, Vol 30, No. 3, March 2006, pages 247-253) was published by Karimi et all: "Conversion of rice straw to sugars by dilute-acid hydrolysis". Hydrolysis of rice straw by dilute sulphuric acid at high temperature and pressure was investigated in one and two stages. The hydrolyses were carried out in a 10-l reactor, where the hydrolysis retention time (3-10 min), pressure (10-35 bar) and acid concentration (0-1%) were examined. Optimisation of first stage hydrolysis is desirable to achieve the highest yield of the sugars from hemicellulose and also as a pre-treatment for enzymatic hydrolysis. The results show the ability of first stage hydrolysis to depolymerise xylan to xylose with a maximum yield of 80.8% at hydrolysis pressure of 15 bar, 10 min retention time and 0.5% acid concentration. However, the yield of glucose from glucan is relatively low in first stage hydrolysis at a maximum of 25.8%. The solid residuals were subjected to further dilute-acid hydrolysis in this study. This second-stage hydrolysis without addition of the acid could not increase the yield of glucose from glucan beyond 26.6%. On the other hand, the best results of the hydrolysis were achieved, when 0.5% sulfuric acid was added prior to each stage In two-stage hydrolysis. The best results of the second stage of the hydrolysis were achieved at the hydrolysis pressure and the retention time of 30 bar and 3 min in the second stage hydrolysis, where a total of 78.9% of xylan and 46.6% of glucan were converted to xylose and glucose, respectively in the two stages. Formation of furfural and HMF were functions of the hydrolysis pressure, acid concentration, and retention time, whereas the concentration of acetic acid was almost constant at pressure of higher than 10 bar and a total retention time of 10 min.

WO 96/25553 (corresponding with CZ 281 504 B6) is mentioned a method of processing of lignocellulose material by continuous pressure hydrolysis and corresponding equipment. The invention relates to a method and an equipment for the processing of lignocellulose materials by continuous pressure hydrolysis, optionally in the presence of inorganic acid, by consequent expansion and separation of the hydrolysate and gaseous phase, wherein disintegrated raw material is wetted by pressure water of the temperature 170 to 200°C at the ratio water to dry matter 0,5 to 1:1 from the mixture obtained excessive water is removed by pressing to the ratio of dry matter to water 1:0,3 to 0,5 consequently with simultaneous feed of pressure water of the temperature 170 to 200°C in the ratio to dry matter 1:2,5 to 4 it is hydrolysed at the temperature 160 to 230°C and pressure 0,6 to 2,8 MPa for 3 to 18 minutes, while hydrolysis proceeds with simultaneous uniform advancement of solid and liquid phases, after termination of hydrolysis the material is expanded in two stages giving rise to gaseous phase and hydrolysate, gaseous phase contains furfural, methanol and acetic acid, hydrolysate contains cellulose, lignin and water, gaseous phase is rectified and separated to furfural mixture and mixture of acetic acid, formic acid and water, water is removed from hydrolysate by pressing and solid remnant is extracted by solvent from group formed by ethanol or acetone, lignin is extracted to the solvent and after evaporation of the solvent reactive lignin is obtained, cellulose remains in solid phase, furfural obtained from the gaseous phase is purified by other distillation and pure furfural may be further treated to furane The invention also mentions, that after hydrolysis creating suspension shall expand to the middle-pressure and normal atmospheric pressure at least in two stages.

The improvement of this solution presents WO 2006/024 242 A1 (corresponding with EP 1 836 181 B1 and CZ 294 398 B6), which describes method and device for the continuous processing of renewable raw materials. By means of pressure hydrolysis of lignocellulose material and subsequent enzymatic hydrolysed residues is obtained glucose, to which starch raw materials are added and all of which undergoes amylolytic cleavage and spirituous fermentation. The lignocellulose raw material is continuously hydrolysed, the hydrolysed material is expanded In two stages, when the vapour phase and hydrolysate solution are produced. In the vapour phase there is furfural, methanol and acetic acid, the hydrolysate solution contains sugars, lignin with residual cellulose and water. The hydrolysate solution is separated into the solution of sugar and solid un-reacted phase by pressuring, the solid phase is exposed to cellulase enzymes, by the process of which soluble glucose and insoluble lignin are produced, the lignin is separated. The solution of glucose is added to the solution of sugar from the hydrolysis, to the solution of sugars is continuously added starch material and everything undergoes amylolytic hydrolysis, after which the solid particles are separated and returned to thermo-pressure hydrolysis, the solution of glucose is pumped for fermentation, where the glucose ferments to ethanol, the yeast cells are separated and ethanol is distilled off. The device, comprises the filing unit, hydrolysers, expanders, distilling and rectifying column, is represented in that continuous worm pressure filling unit, consists of segments formed by the body, with single-threated conveyor worms place on the shaft. Between the worms, there is at least one steam ring, which conically widens on the input side of raw material. In the outlet filler with the reducing part leading into first hydrolyse a pressure identical to that of the first hydrolyser is achieved. The last hydrolyser is connected to the rectifying column for furfural and to section of the cellulolytic enzyme hydrolysis, amylolytic cleavage, and ethanol fermentation.

Continual method of ligno-cellulosic material hydrolysis is not used industrially until now. Very short reaction periods, to ensure quick heating of raw material mixture, and the heat regeneration can be very difficult problems, when there is necessary to ensure economic effectivity. Next disadvantage is the uncomplex usage of all products which are created with the help of these methods.

Very important condition of hydrolysic processes for ligno-cellulotic materials working up is the ensuring of operational continuity of production, universal equipment for various kinds of ligno-cellulotic materials, optimalization of hydrolysis process parameters, complex and the economical profitability of products created with the help of this type of hydrolysis, and their next utilization.

### Summary of the Invention

Above mentioned disadvantages of todays'hydrolytic methods of monosaccharides (glucose), ethanol, furfural, furane, clean lignin, acetic-acid and formic-acid, alcohol fermentation residues and carbon oxide, are solved with the help of technologic and economic method of continual pressure hydrolysis of ligno-cellulosic materials, according to the method of claim 1 and the equipment according to claims 2-7.

Also disclosed are the following methods and equipments:
A method, which is based on the special technology, i.e. the ligno-cellulosic material,
crushed to small particles 10 till 30 mm, shall go through the feeding endless screw press. Simultaneously, the material shall be moistened up a little by technological water in the rate of 0.3 till 10% of the entering material mass. Whole volume of material shall be heated to temperature 80 till 90 °C, and material, which has been cut up and heated, shall be continually hydrolysed with the help of the technological water and vapour. If there is applied two-stage hydrolyse:
   At first stage the hemicelluloses will be cracked to pentoses when the temperature reached 160 till 185°C and
   pressure 0.6 till 1.0 MPa, after 8 till 10 minutes the hydrolysed suspension will be separated in the squeezing and conversion press to fluid phase containing pentoses,
   which shall go on to the expansion, and eventually to the next working up. Solid unreacted ligno-cellulosic phase shall be forced through the conversion press into the second stage of hydrolysis, in -which the pressure hot water (temp. 200 till 240 °C and pressure 1.6 till 3.3 MPa, and hydro-module 1:2 till 1:3.5) shall be acting 8 till 10 minutes, and together with water the dilluted solution of anorganic acid at the rate to suspension 0.1 till 1%, it shall be alternatively sprinklig. The hydrolysis is going on, whilst the solid and fluid phase is shifted as equilibrilium. Phosphor-acid or
   hydrochlorid-acid, eventually some other acid shall be feeded by a pump, so that the treatment of pH and its acidity shall be prepared, into feeding piping in front of hydrolyser. All vapour in the hydrolytic system will be condensated up, and it will make the raw material hot.
   Evaporation heat will cover also the heat losses through the metal jacket of the second hydrolyser. There shall occur the hydrolytic cracking of hemicellulosis onto pentosans, and the mixture containing furfural, acetic-acid, formic-acid, methanol, hydrolytic saccharides as glukose, and the cracked ligno-cellulosic phase, which is unreacted yet.

At occasion of single-stage hydrolysis, the solution of created substances mixtures, which have the hydromodule 1:4 till 1:5 shall be led in the hydrolysers, during its movement in hydrolysers for the period 10 till 12 minutes, with the help of temperature 210 till 240 °C, and the pressure 1.8 till 3.3 MPa. During this hydrolysis the cracking of pentoses onto pentosans shall be carried out, and by the dehydratation onto furfural, acetic-acid, formic-acid, methanol, and the cracking of ligno-cellulosic complex structures onto the hydrolytic saccharides shall be done. This suspension shall go on between the hydrolysers cross the overflow pressure pipe into the next hydrolytic section to be subjected to the final hydrolysis, and two or three phases of expansion to the atmospheric pressure shall follow, what will be the cause of evaporation of fluid solution containing furfural, acetic-acid, formic-acid, methanol and water. Part of vapours shall be taken away with the help of innert gases and the high-pressure expander slide-valve into heat exchanger. Mixture of hydrolytic saccharides, and the unreacted cracked ligno-cellulosic phase, which is going through press equipment where the separation of the hydrolytic saccharide solution and the unreacted solid ligno-cellulosic phase occurs. This unreacted phase shall go for the enzym hydrolysis, and it will be worked up to glucose and clean lignin.

Next advantageously solved technological cycle of continual hydrolysis, which belongs to equipment according to the invention, is the furfural separation cycle. Vapour mixture containing water, fural, methanol, acetic-acid and formic-acid shall go from the hydrolytic cycle into the separation cycle. The mixture shall be continually feeding into the rectification column. From the rectifier it shall go away as the distilled mixture containing furfural, methanol and water, and the mixture of acids and water as distillating rest. The distillate shall be taken away into decantation vessel after its cooling, and in this vessel shall be separated as the heterogenic mixture to two layers. Top layer containing about 8 wt.% of furfural shall be returned into decantation vessel. The bottom layer containing 92 wt.% of furfural shall be kept in the supply tank. Both mixtures shall be separated by the rectification distillation in the column. Methanol which is created as the distillate shall be taken away and supplied in supply tank, and the mixture of furfural, methanol and water as distilling rest shall go away, water shall be turned back to supply tank.

During separating of mixture from the supply tank, which contains first of all furfural, the water contaminated by furfural and methanol shall be taken away from rectifier as distillate. This flow shall be taken back into the decanter. Fural in concentration of 99.67% shall be taken as distillation rest to the supply tank. In bottom part of rectifier are deposited the heavy distilling fractions according to the quality of the worked up raw material located in the hydrolytic unit. These pitches shall be time to time remove from the column, i.e. they shall be taken away to supply tank.

At occation of the direct production of furan from furfural, which may reach the volume concentration 99.67%, without simultaneous extraction of furfurylalcohol, fural shall be charged into the autoclave where shall be added catalyst (CaO, CaCO₃, MnCrO₂, or ZnCrO₂). After covering the autoclave shall be heated to temperature 400 °C. There will be separated furan, which shall be cooled in heat exchanger, and it will be deposited in tank. Also carbon oxide shall be taken away from autoclave, and it is liquidated by oxidizing combustion in the combustion chamber. Production process of furan gaining will not be carrying out as continual process, because there will go the reaction in the autoclave at high temperature and pressure.

Distilling rest from the rectifier containing acetic-acid, formic-acid and water shall be taken away to supply tank and from that shall be continually sprinkled into top part of extraction column. Ethylacetate with water which can extract the acids in water shall be transported from the supply tank to the bottom part of column. Two flows shall be taken away from the extraction column. Top flow contains the water solution of acids and ethylacetate which shall be transported onto rectifier, where the water solution of ethylacetate shall be separated from the mixture, and it shall go to supply tank and there shall be recycled. Distilling rest contains the water solution of acids and rest of ethylacetate, and it shall be taken away into supply tank. Bottom flow from the extracting column, containing the rest amount of acids and ethylacetate dilluted in water, shall be taken away into the supply tank. Intermediate products in the supply tanks shall be worked up as follows: both mixtures shall be entering into rectifier alternatively. Water as the distillate with low volume of ethylacetate shall be taken away at the occasion of the supply tank mixture working up. This mixture shall be sprayed again into the rectifier. Mixture of clean acids as the distilling rest shall be taken away from the column, and it shall go to the supply tank. Solution of the hydrolysate containing hydrolytic saccharides, the solid ligno-cellulosic phase as fibrous material and water shall be continually taken away from the bottom part of expander, cross a rotating shutter. From the supply tank where the solution is mixing, it is pumping onto the filtration press or centrifugal separator. After pressing there shall be gained the solution of sugar and water, and the solid fibrous ligno-cellulosic rests, which shall turned back into thermo-pressure hydrolysis for the final hydrolysis, or these rests shall be hydrolysed by the enzymes cellobiose and cellulast. These combined process provissions will ensure the high profitability of fermented sugars.

Solution of saccharides from the hydrolysis shall be continually led into the amylaceous material and the mixture shall be subjected to the amylotic hydrolysis, and the rest solid parts containing non-starch grain composition will be separated from resulted reacting mixture. Rest solid parts shall be turned back to the thermo-presure hydrolysis. Glucose solution, first of all treated for certain pH factor, after addition of salt and nutritions, and after treatment of glucose concentration eventual dillution with unthickened distillery slops from the vapour column, cross heat exchanger, into the fermentor. New fermentation can be run very quickly as the separated yeast shall be turned back to the feeding fermentation, or 20 till 30% of fermentor volume may be kept in the fermentor as the starter of fermentation. The solution created by glucose fermentation when it will be changed to ethanol, and after the yeast separation, shall be pumped onto distillation. About 90% of ethanol in concentration nearly 40% shall be taken away in the form of vapour from distillation column into rectificating column, and the part of distillery slops shall be turned back into the fermentation, where it will dillute the saccharides solution to needed concentration, and unused distillery slops shall go on to the evaporator. Distillery slops can be thickened to the required concentration of dry substance. This whole process has in industrial conditions high profitability, and distillation effectivity is approximately 99.5%.

Compactability and linkability of all working up steps, i.e. hydrolysis, fermentation, distillation and rectification, and together with steps for the usage of side products as fural, lignin, distillation slops, yeasts and carbon oxide can ensure the automatization of production process, and reach the favourable system of renewable sources working up without any waste.

Temperature of solution entering from the thermo-pressure hydrolysis is using for the starch gelation which is being added into the solution, with eventual final heating of technological water or vapour. After the starch changes to starch jelly and adjusting of optimal water temperature suitable for the thermo-stable amylases, the process of starch cracking to glucose is going very quickly. The advantage of this method is, that the heat of hydrolytic solution can be used.

Heat of glucose solution, and heat of distillation slops can be used for preheating of water spray into vapour column. Heat of exhausting water shall be used for the improvement of energetic ballance of thermo-pressure hydrolysis. Waste heat can be used in working sets very effectively. Waste water, in whole measure, shall be turned back into process, except water in moistured materials and washing water. Low concentrated acid shall be used for hydrolysis process in volume of 0.3 till 1% of mass. Very useful can be the usage of phosphor-acid, of which salts serve during the fermentation as the nutrients.

Advantage of this method and equipment is first of all the fact that the hydrolytic fermentation process is working up the entering material completely to the saleable products as fural, furylalcohol, furane, lignin, ethanol, acetic-acid, formic-acid, carbon oxide and distillery slops with yeasts.

Next advantage of the production of all above mentioned products is the solution into a compact production unit, and this method is using the ligno-cellulosic and amylaceous materials, and the agricultural vegetable can be worked up completely in wasteless process. Waste waters are the only waste.

Technical and technological core of the equipment is the complex of feeding, pressuring, conversion and transmittal machinery, and hydrolytic, decompressing and other equipments which enable the transport of entering raw materials and suspenses with the help of continually flowing and mixing operation in hydrolysers, and with the help of needed range of temperature, pressure and delay period in the hydrolytic part of ligno-cellulosic materials, where the starch is working up in the continually linkage. Process of the hydrolytic fermentation technology of bio-ethanol production has very important feature for the protection of environment, i.e. this production is solved by a compact production unit, working up the renewable sources of materials, and without any significant waste.

The equipment for this production method can be completely produced in machinery factories of the middle production volume without any dependance on the import. This method establishment enables the production of bio-ethanol and several other products from saccharidic sources, i.e. ligno-cellulosic materials, with high effectivity 75 till 85%, and with other advatages as follows:
- revitalization of agricultural enterprices, more working occasions
- usage of bio-ethanol which has been produced by designed technology
- economical effectivity
- ability for competition all over the world
- reducing of imported mineral fuels (oil)
- technologic universality
- no harmful impact to environments
- utilization of renewable sources
- variability of sources
- possibility of new technologies and know-how export

### Brief Description of the Drawings

The invention shall be more detailed clarified with the help of drawings as examples where graphic description is given:

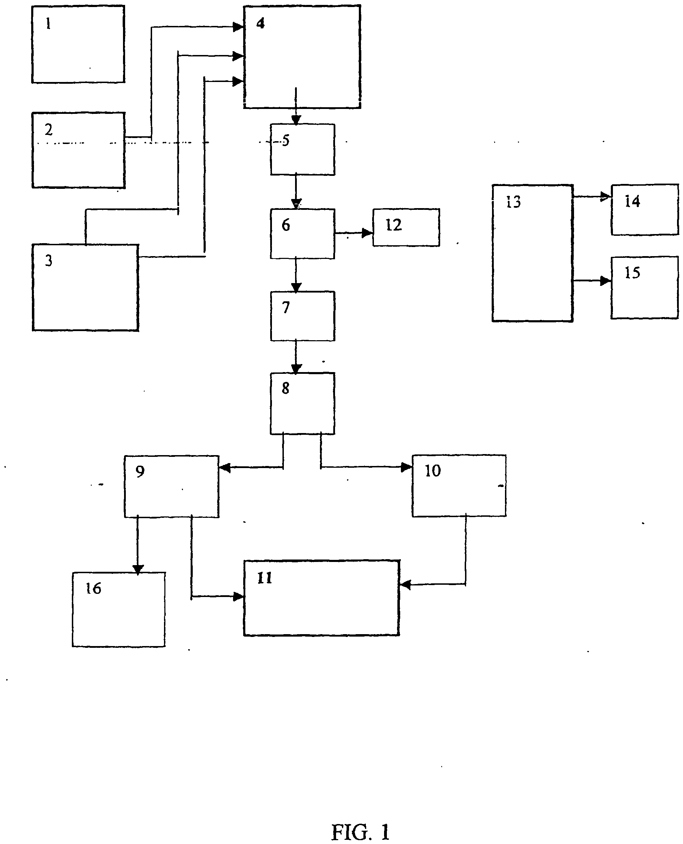

- Fig. 1 -: block scheme of pressure and enzymatic hydrolysis, and separation
- Fig. 2 -: block scheme of fural mixture separation from vapour phase of hydrolysis
- Fig. 3 -: block scheme of fural working up to furane
- Fig. 4 -: block scheme of acetic-acid and formic-acid and water separation
- Fig. 5 -: block scheme of fermentation, and ethanol separation from glucose gained by hydrolysis of ligno-cellulosic materials and starch
- Fig. 6 -: decompressing, conversing and transmitting endless screw into counter pressure

### Description of the Preffered Embodiment

Ligno-cellulosic material, after its preadaptation onto small parts 10 till 30 mm, shall be compressed in the feeding endless screw press, and during this operation the material shall be moistured a little by technologic water in volume of 8% of entering material mass, which shall be compressed step by step, and simultaneously heated to the temperature of 85 °C. Crushed and heated material shall be continually hydrolysed with the help of technologic water and vapour. When the two-stage hydrolysis is applied, the hemicelluloses will be cracked to pentoses in first stage at temperature 160 till 185 °C-and pressure of 0.8 MPa, with the help of delay period 8 till 10 minutes and folowing decompressing in press, where the hydrolysed suspension shall be separated onto the fluid phase containing pentoses, and it shall go on to the expansion and eventually to next working up step. Solid unreacted ligno-cellulosic phase shall be pushed through the conversion and transmitting press into second stage of hydrolysis. Pressure hot water with temperature 220 °C, and pressure of 2.8 MPa, and hydro-module 1:3 during the exposing time, i.e. delay period 8 till 10 minutes for ligno-cellulosic suspension exposition, and together with the water, the dilluted solution of phosphoric-acid in the rate 0.8 to the suspension shall be feeding in two phases, and the hydrolysis shall go on with the help of simultaneous continual shift of the solid and fluid phase. Phosphoric-acid or chlorhydrogenic-acid, eventually some other acid shall be feeding with the pump for pH treatment and acidity treatment into feedeng pipe in front of the hydrolyser. All vapour shall be condensing in the hydrolytic system, and the passaging material shall be heated. Condensing heat shall cover also the heat losses cross the coat of second hydrolyser. Catalytic reaction of the acid, pressure and temperature shall ensure the hydrolytic cracking of hemicelluloses to the pentosans, and the mixture containing furfural, acetic-acid, formic-acid, methanol, hydrolytic saccharides, i.e. glucose and the cracked unreacted ligno-cellulosic phase shall be created.

During one-stage hydrolysis the solution of done mixture of created substances shall be led in the hydrolyser for the delay period of 10 till 12 minutes, with the help of high temperature 210 till 240 °C and the pressure of 2.2 MPa, and hydro-module 1:5. This temperature and pressure in the acidic medium shall cause the cracking of pentoses to pentosans, and the dehydratation to furfural, acetic-acid and formic-acid, methanol, and the cracking of ligno-cellulosic complex to the hydrolytic saccharides. The suspension shall be led between both hydrolysers, cross the out-flow pressure pipe, into next hydrolytic section to the final hydrolysis. As a next step the suspension shall be kept to expand to the atmospheric pressure, and the evaporization of fluid solution part containing furfural, acetic-acid, formic-acid, methanol and water, will occur. Part of vapours shall be led away by the help of high pressure shutter into the heat exchanger. The mixture of hydrolytic saccharides and unreacted cracked ligno-cellulosic phase shall be kept in the fluid part which is transmitted from the bottom part of expander. Unreacted cracked ligno-cellulosic phase shall be pushed through the press where the separation of hydrolytic saccharides and unreacted solid ligno-cellulotic phase will occure, which shall be worked up to the glucose and clean lignin with the help of enzymatic hydrolysis, which will follow.

During the furfural separation from the hydrolytic cycle, the vapour phase containing the mixture of water, fural, methanol, acetic-acid and formic-acid is entering the separation cycle. Then the mixture shall be continually feeding into the rectification column, from where the mixture of furfural, methanol and water as the distillate, and the mixture of acids and water as distillating rest shall go away. Distillate shall be taken away into decanting vessel after its cooling, where it shall be separated as the heterogenous mixture to two layers. Top layer contains approximately 8 wt.% of furfural, and it shall be leading back into decanting vessel. Bottom layer contains 92% of furfural, and it shall be deposited in the container. Both mixtures shall be separated by rectification distillation in the column. Methanol which shall be taken away as the distillate from the tank during the separation of mixture, and it shall be deposited in a container, and the mixture of furfural and methanol with water. Water shall be turned back into container. At the occasion of mixture separating from the container, which contains mainly furfural, the water contaminated by furfural and methanol shall be taken away as the distillate from rectification column. This flow shall be led back to decanter. Furfural of concentration 99.67% shall be taken away into a container as the distillating rest. Heavy distillation fractions are deposited in the bottom part of column, according to the quality of feeding raw material in the hydrolytic line itself. These pitches shall be removed from the column time to time, i.e. they shall be taken away and deposited into the tank.

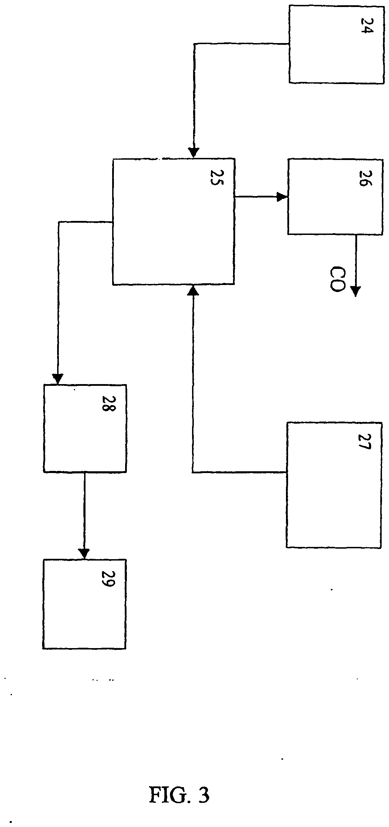

As apparent from Fig. 3, at the occation of direct furan production from furfural with the volume concentration of 99.67%, without simultaneous gaining of furfurylalcohol, the fural shall be charged from a tank into the autoclave including catalyst, in this execution example the catalyst shall be calcium oxide CaO. Autoclave shall be heated to the temperature of 400 °C after its covering. There are separated out the furan, which shall be cooled in the heat exchanger, and it shall be deposited in tank. Also carbon oxide shall go away from the autoclave which shall be liquidated by combustion in the combustion chamber. Production proces of furan creating cannot be realized continually with regard to very high temperature and pressure, at which the reaction in autoclave is going on.

Distillation rest from the rectification column, containing the acetic-acid and formic-acid with water, shall be led into a container, from where it shall be continually sprayed into the top part of extraction column. Ethylacetate with water, which can extract the acids in water, shall be transported from the container into bottom part of the column. There are two flows which shall be taken away from the extraction column:
The top flow containing water solution of acids and
ethyacetate which shall be transported into rectification column for separation of water solution of ethylacetate,
and the ethylacetate rest, and it shall be taken away into the container, and from this container it shall be recycled. Distillation rest contains the water solution of acids, and rest of ethylacetate, and it shall be taken away into container.

The bottom flow from extraction column containing rest amount of the acids and ethylacetate dilluted in water shall be taken away into another container.

Intermediate products in the containers shall be worked up as follows. Both mixtures shall be entering alternatively into the rectification column. The water with low volume of ethyacetate shall be taken away from the column at the occasion of working up of mixture from the container. This mixture shall be sprayed into the rectification column. Mixture of clean acids from the column shall be taken away as the distillation rest, and it shall go into container.

Solution of hydrolysate containing hydrolytic saccharides, defibered solid ligno-cellulotic phase and water shall be continually taken away from the bottom part of expanders, cross rotary shutter, into the moving and mixing container, from where it shall be pumped onto the fitration press or centrifuge. There can be gained the solution of saccharides and water, and solid defibered ligno-cellulotic rests, which shall be turned back to the thermo-pressure hydrolysis towards the final hydrolysis, or the enzymes shall be hydrolysed by celobiose and cellulast. These very easy combinable processing provisions can ensure high efficiency of the fermentable saccharides production.

Solution of saccharides from the hydrolysis shall be continually led into amylaceous material, and both parts shall be subjected to the amylolytic hydrolysis together, and at this occasion the solid parts containing non-amylaceous rate of grains shall be separated, and these parts shall be turned back into the thermo-pressure hydrolysis. Solution of glucose, after the treatment of pH and addition of salts and nutritions, and after the regulation of glucose concentration by eventual dillution with the unthickened distillery slops from the mash column, shall be fed cross the heat exchanger into fermentation column. Feeding fermentation with turning back of separated yields may be very suitable, otherwise 20 till 30% of the fermentor volume can be kept in fermenting column, as the starter for next fermentation, and the next fermentation will go ahead very quickly as well. Solution from the fermentor shall be pumped, after glucose fermentation to ethanol, and after yields separation, onto the distillation. Approximately 90% of ethanol with concentration about 40% shall go away as vapours into the rectification column, and at this occasion the part of distillery slops shall be turned back into fermentation, where the saccharide solution shall be diluted to needed concentration, and the unused part of distillery slops shall go on onto evapouring unit. Distillery slops can be thickened in evapourator to the required dry matter concentration. Whole process has high efficiency in the industrial conditions, and its distilling effectivity can be approximately 99.5%.

For example the wheat straw at the occasion of single-stage method, after its treatment and crushing to the particles of size 10 till 20 mm, shall be compressed in feeding press, and the material shall be simultaneously moistured by technological water with temperature of 30 °C, the amount of which shall be 8% of entering mass. Raw material shall be hydrolysed with the help of temperature 220 °C and pressure 1.6 MPa, and during the period 10 minutes in two hydrolysers, the hydromodule shall be 1:4, and the hydrolysis is passing at simultaneous advancement of solid and fluid phase. After finishing of the hydrolysis, material shall be expanded in two stages, and the vapour phase and hydrolysate origins. Vapour phase contains furfural, methanol and lower organic acids, the hydrolasate contains hydrolytic saccharides, lignin and water. Vapour phase shall be subjected to the rectification and there it is separated to the furfural mixture and mixture of acetic-acid, formic-acid and water. The hydrolysate shall be taken off the hydrolytic saccharides solution, and the unreacted solid ligno-cellulotic phase shall be led to the enzyme hydrolysis at which the degradation of solid phase to glucose occurs, and the separation of clean lignin, which shall be taken away to a container. Glucose from the first stage, and from the enzyme hydrolysis and starch shall go ahead into common container of saccharides to the preparation of fermenting process, next to the distillation and to dewatering of ethanol.

In the case of rape straw the whole process shall be the same, but the difference refers to parameters during working up: temperature 230°C and pressure 2.3 MPa, delay period 12 minutes in the hydrolysers, the hydromodule shall be 1:4.5.

Equipment for the implementation of this method consists from equipment for the entering raw material preparation, containers, feeding pressure equipment, and at least two hydrolysers where the last one is interconnected cross the middle-pressure expander and low-pressure expander with the moving and mixing container of hydrolytic product, and the top part of middle-pressure expander and low-pressure expander is interconnected with the top part of rectification column of furfural and with the furfural container. The equipment further contains the fermenting vessels, and the distillating column for prefermented mash containing ethanol, and the two-stage hydrolysis has between first and second hydrolysers the decompressing, conversing and transmitting press, which keeps different pressures between both hydrolysers.

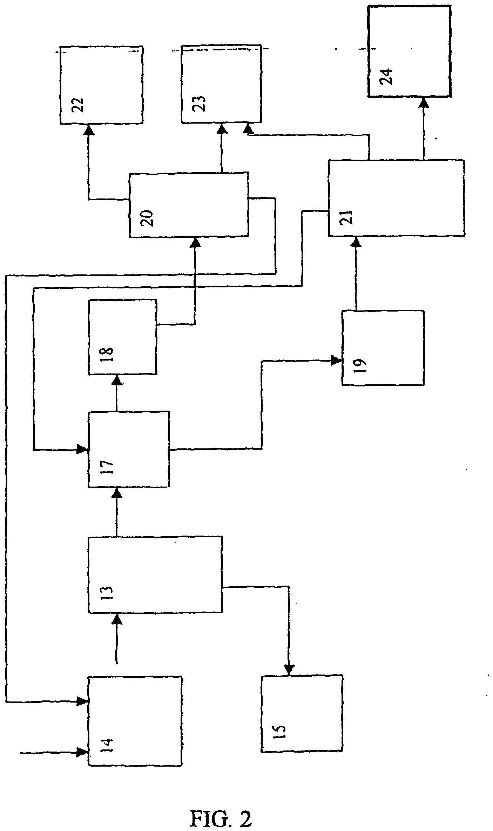

As apparent from Fig. 2 the container of furfural mixture 14 is connected to the rectification column 13, which is connected with the decanter 17 by piping for methanol and water, the decanter is connected by pipe with the container 18 for the low-percentage furfural mixed with water , and with the furfural container 19. Container 18 for low-percentage furfural with water is connected with the methanol column 20, and this column is connected to the methanol container 22 and 23, bottom part of the methanol column 20 is connected with the furfural mixture container 14. Furfural container 19 is connected with the vacuum rectification column 21, its top part is connected to decanter 17. and its bottom part is connected with the clean furfural container (of volume concentration 99.67%) 24. the rectification column is further connected with the methanol container 23.

Equipment for the working up of furfural to furane, as apparent from Fig. 3, contains the clean furfural container 24, which is connected with the pressure melting furnace 25, the container 27 of catalyst is connected to the furnace, and it is also connected with the oxidizing furnace 26 by piping for carbon oxide. Bottom part of the pressure melting furnace 25 is connected, cross an intermediate container 28, with the container 29 for furan .

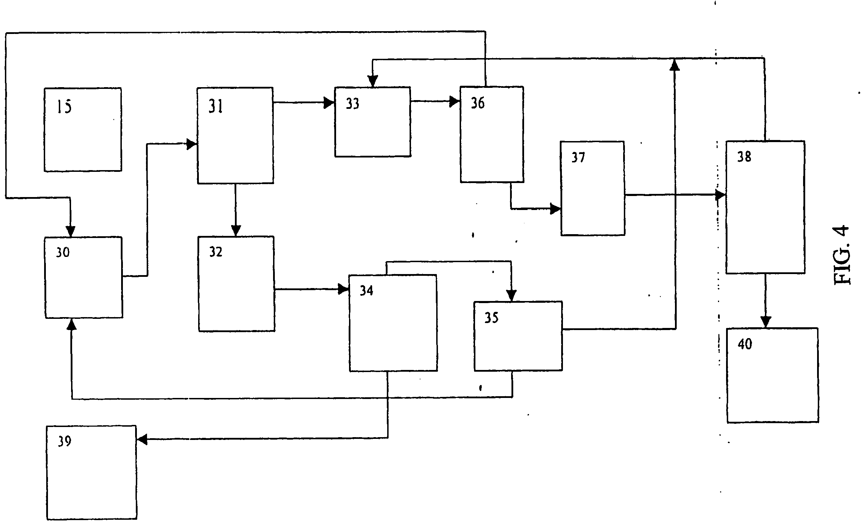

Equipment for the acetic-acid, formic-acid and water separation as apparent from Fig. 4, is combined by the acids container 15 and container 30 for ethylacetate, which are connected to the extraction column. Outflow pipe from this extraction column 31 is led into the waste water container 32, this container is connected with the rectification column 34 of waste water, its bottom part is connected with the waste water container 39, its top part is connected with decanter 35, which is connected with the ethylacetate container 30. Rectification column 31 is connected with the container 33 of acid, ethylacetat and water mixture, which is connected with the rectification column 36 of ethylacetate, its top part is connected with the container 30 for ethylactate, and its bottom part is connected with the container 37 of acids, this one is connected to the rectification column 38 of acids , its top part is connected with the acid mixture container 33, and bottom part is connected with the clean acids container 40.

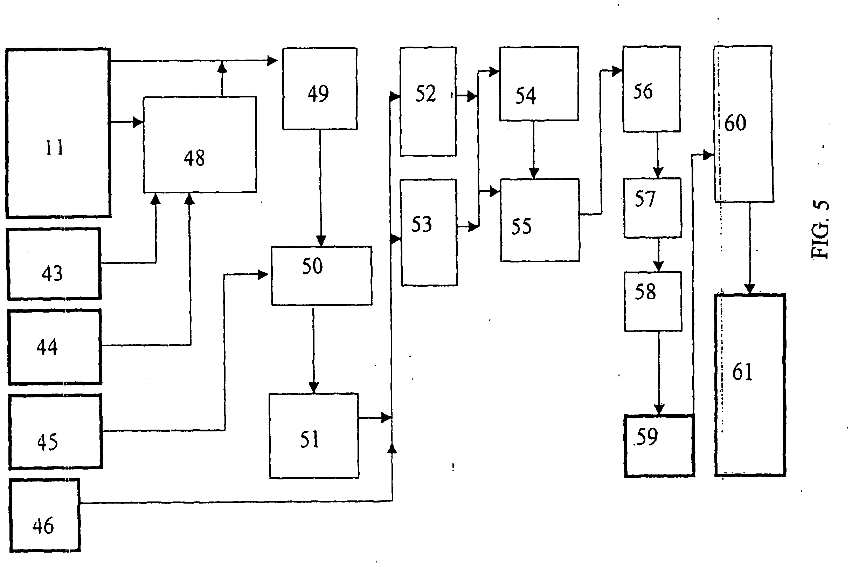

Equipment for ethanol fermentation and separation, as apparent from Fig. 5 is assembled from the saccharide solution container 11 creating in thermo-pressure and enzyme hydrolysis, which is directly connected to the heated liquefying tank 49, and as the hot water container 44 and as the defibered amylaceous raw material 43 is interconnected with the apparatus for suspension preparation 48. This apparatus 48 is connected with the heated liquefying tank 49 which is connected with the saccharification tank 50, which is further connected ith the tank 45 for the preparation of amylolytic enzymes and with the pump of sweet mash, which is connected with the fermentors 52 and 53, which are connected with the vessel for preparation of seed yeast (starter) 46 and with the yeast separation vessel 54 and feeding pump 55 of the slime pulp column 57, which is connected cross the slime pulp column cooler 57 and the crude ethanol cooler 58 with the crude ethanol container 59, which is interconnected, cross the rectification column and dewatering equipment 60, with the waterless ethanol container 61.

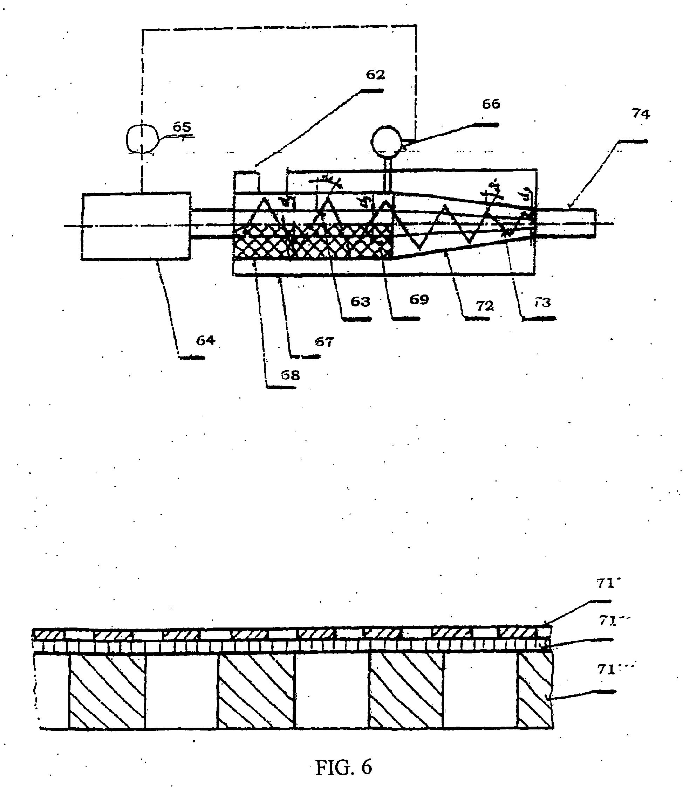

Equipment of the decompressing, conversing and tranmitting press into counter-pressure, graphically descripted in Fig. 6, which is used at the case of two-stage hydrolysis, compounds from four zones. First zone is made by the feeder 63, which is represented by the cylindric body 68 and cylindric spindle 69 with a constant lead of spindle worm. Cylindric body 68 is provided by the sandwich perforation under the axis of revolution 71, second zone is made also by cylindric body and cylindric spindle with constant lead of worm and with the volume contraction of thread profile, and cylindric body is provided by sandwich perforation as well. Third zone is created by conical body 72 and conical spindle 73 with the decreasing lead of helix, and the conical body is furnished by the system of conical areas and small radial channels. Fourth zone is created by the pressure feeding head 74 of cylindric shape, and the driving gear unit 64 with transmission interconnected with the spindle. Driving gear unit is provided by a switching ampermeter 65 eventually interconnected with the fluid feeding equipment 66.

### Field of the Application

The invention can be utilized:
The main product of continual production process is ethanol of concentration more than 98% of mass. Several possible variants are important for its utilization:
   A) Ethanol can be added into motor fuels
   B) Ethanol itself can be used for the energy production
   C) Other utilities

A. Variant "A" appears as the most advantageous utility of ethanol, so as additive to the motor fuels. Possibility of the direct ethanol addition in rate 10 till 30% into motor fuels without any necessary adjustment or change of motor construction. Effect of the addition may be apparent in the lowering of harmful exhaust emissions (namely CO).

B. Variant B. Ethanol itself usage for the energy production may be designed in case, if process of ethanol addition into motor fuels should be somewhere very problematic. This variant is connected with the idea of electric power or heat energy production, and according to the needs in individual region. This variant would make possible to add ethanol, industrially produced, to combustion process, what would increase the heating capacity of solid or gas fuel, for example the entering of ethanol with the help of spray nozzle into the combustion chamber with burners.

C. This variant has several partly possibilities, which are individually limited by capabilities of customers. One of this partly variant is the usage of some part of ethanol industrial production in the industry of paints and lacquers as a solvent. Another possibility is the treatment of some production part of ethanol for chemical and food industry.

Further important products and their utilization:
Further most important products of ethanol industrial production are furaldehyd, lignin, acetic-acid, formic-acid, limited amount of methanol, carbon oxide, distillery slops and yeasts.

Lignin is well salable raw material, required namely as the part of filling materials in the rubber industry, and it has very favourable features for the quality of produced materials (mainly for tyre production).

Furaldehyd, acetic-acid, and formic-acid are the commodities, which can be well sold on the chemical product markets. As lignin, all of them are well salable products, which can be produced in needed quality, and have good impact to economy of ethanol production.

Methanol is well salable on chemical product markets, as it can be reasonably used in the industry of motor fuels.

Carbon oxide (CO₂) is taken away relatively in huge amount and quality (it is practically clean exit from the biologic process fulfilling all demands of food industry) . There is obvious, that its taking away can create the important part of production.

Distillery slops and yeasts can be used in the agriculture. There is suggested to use them alternatively as the raw material for biologic gas production.

### List of Reference Signs

- 1: Preparation of raw materials
- 2: Raw materials container
- 3: Heating cycle
- 4: Feeding equipment with hydrolysers
- 5: Pressure shutter
- 6: Expanders
- 7: Fluid product container
- 8: Press - separator of solution and solid rests
- 9: Enzyme hydrolysis
- 10: Solution of hydrolytic saccharides, i.e. glucose
- 11: Container for monosaccharides created at pressure and enzyme hydrolysis
- 12: Fural container
- 13: Rectification column
- 14: Fural container
- 15: Container of acids
- 16: Container of lignin
- 17: Decanter
- 18: Container of low-parcentage fural
- 19: Fural container
- 20: Methanol column
- 21: Vacuum rectification column
- 22: Container of methanol
- 23: Container of methanol fraction
- 24: Container of clean fural of volume concentration 99.67%
- 25: Pressure melting furnace
- 26: Furnace for CO oxidation
- 27: Container of catalyst
- 28: Cooler / heat exchanger
- 29: Furan container
- 30: Ethylacetate container
- 31: Extraction column
- 32: Waste water container
- 33: Container for mixture of acids, ethylacetate and water
- 34: Rectification column of waste water
- 35: Decanter
- 36: Rectification column of ethylacetate
- 37: Container of acids
- 38: Rectification column of acids
- 39: Waste water container
- 40: Container of clean acids
- 41: Containers for grain raw material
- 42: Tank for lime milk
- 43: Hammer mill for crushing of amylaceous raw material
- 44: Hot water tank (hot-well)
- 45: Enzyme tank
- 46: Fermenting tank
- 47: Pump of seed yeast (starter)
- 48: Preparation of suspension
- 49: Heated liquefying tank
- 50: Saccharification tank -
- 51: Cooler and pump of sweet mash
- 52: Fermenting tank - fermentor
- 53: Fermenting tank - fermentor
- 54: Yeast separation
- 55: Feeding pump of slime pulp
- 56: Slime pulp intensificating column
- 57: Cooler of slime pulp column
- 58: Cooler of crude ethanol
- 59: Crude ethanol container
- 60: Rectification and dewatering of ethanol
- 61: Container of waterless ethanol
- 62: Feeding
- 63: Feeder consists of cylindric body
- 64: Driving gear unit
- 65: Ampermeter
- 66: Injection equipment
- 67: Cylindric coat / channel
- 68: Cylindric body
- 69: Cylindric spindle
- 70: Sandwich perforation
- 71': Perforated metal sheet
- 71": Filtration netting
- 71"': Outer perforated coat
- 72: Conical body
- 73: Conical spindle
- 74: Pressure feeding head

## Claims

1. Method for production of monosaccharides, ethanol, furfural, furane, methanol, acetic-acid, formic-acid, and lignin produced from renewable raw polymer materials by the continual thermo-pressure hydrolysis, combined with enzyme hydrolysis, wherein:
- the hydrolysed suspension is expanded to the middle-pressure and normal atmospheric pressure in at least two stages, whereby it is separated into a vapour phase containing besides the water also furfural, methanol, acetic-acid, formic-acid, and into a fluid phase containing the water solution of hydrolytic saccharides and other dilutable matters, and into an unreacted solid ligno-cellulosic phase,
- the said unreacted solid ligno-cellulosic phase is separated by pressing and/or filtration, and after addition of water it is subjected to the action of cellulosic enzymes, and at this reaction monosaccharides are formed,
- the said monosaccharides are subjected to amylolytic hydrolysis after separation of uncracked lignin, together with the treatment of amylaceous materials and a fluid phase, whereafter the saccharide solution is further worked up, and/or whereafter the saccharide solution as sweet mash is fermented to ethanol, which is dehydrated and concentrated after separation of the yeast,
the method being **characterized by** fact that it comprises the following features :
a) the renewable raw material is disintegrated to particles of the size 10 - 30 mm, before being subjected to the continual two stage thermo-pressure hydrolysis,
b) the said disintegrated material is moistened by water spraying at the place of feeding pressure equipment using warm water of 20 - 40°C in the amount of 5 - 10 % of mass of entering material,
c) the continual thermo-pressure hydrolysis is carried out as a two-stage
process, where
at first stage the temperature is 150 - 185 °C and the pressure is 0.6 - 1.0 MPa and at a hydromodule of 1:4,
and at second stage there is sprayed additional pressure warm water of temperature 200 - 240°C and pressure of 1.6 - 3.3 MPa,
and at this occasion hydrochloric-acid or some other suitable acid is simultaneously sprayed in volume 0.1 - 1% related to the suspension with a hydromodule of 1:3 to 1:4,
and the fluid phase is separated after finishing of hydrolysis, and after its thickening it is used as the raw material for fermentation to ethanol or furfural.

2. Equipment for implementation of the method according to claim 1, consisting of equipment (1) for preparation of entering raw materials, containers (2), feeding pressure equipment (4) with at least two hydrolysers, which are connected, through the middle-pressure expander and low-pressure expanders (6), with the moving and mixing container (7) for hydrolytic product, and the top part of middle-pressure expander and low-pressure expander (6) are connected with the top part of the rectification column (13) of furfural, and with the furfural container (14), next this equipment contains the fermentation vessels and distillation column for fermented mash containing ethanol, **characterized by** the fact that the two-stage hydrolysis has between the first and the second hydrolyser (4) the decompressing, conversing and transmitting press (62), which can keep the different pressures between hydrolysers.

3. Equipment according to claim 2, **characterized by** the fact that the container (14) for furfural mixture is connected to the rectification column (13), which is connected with the decanter (17) by the piping for methanol and water, the decanter is connected with the container (18) for low-percentage furfural with water and with the container (19) for furfural, the container for low-percentage furfural with water is connected with the methanol column (20), and this column is connected to the methanol container (22) and (23), the bottom part of the methanol column (20) is connected with the furfural mixture container (14), the furfural container (19) is connected with the vacuum rectification column (21), its top part is connected to the decanter (17), and its bottom part is connected with the clean furfural (volume concentration 99.67%) container (24), and the rectification column (21) is further connected with the container (23) for methanol.

4. Equipment according to claim 3, **characterized by** the fact that the container (24) of clean furfural is connected with the pressure melting furnace (25), to which the container (27) for catalyst is connected, and with the oxidating furnace (26) connected by the piping for carbon oxide, the bottom part of the pressure melting furnace (25) is connected with the furan container (29) through the intermediate container (28) .

5. Equipment according to any of the claims 2 - 3, **characterized by** the fact that the container (15) of acids and container (30) for ethylacetate are connected to the extraction column (31), the outlet from this extraction column (31) leads into the waste water container (32), which is connected with the rectification column (34) of waste water, its bottom part is connected with the waste water container (39), its top part is connected with the decanter (35), which is connected with the ethylacetate container (30), the rectification column is next connected with the container (33) for mixture acids, ethylacetate and water, which is connected with the rectification column (36) of ethylacetate, its top part is connected with container (30) for ethylacetate, and its bottom part is connected with the container (37) of acids, which is connected with the acid mixture container (33), and its bottom part is connected with the container (40) of clean acids.

6. Equipment according to any of the claims 2 - 5, **characterized by** the fact that the container (11) of saccharide solution as a part of thermo-pressure and enzyme hydrolysis is directly connected to the heated liquefying tank (49), and the container (44) of warm water, and container (43) of crushed amylaceous raw material are connected with the apparatus (48) for suspension preparation, which is connected with the heated liquefying tank (49) connected with the saccharifying tank (50), which is connected with the tank (45) for the preparation of amylolytic enzymes and pump (51) of sweet mash, this pump is connected with the fermentors (52) and (53), connected with the vessel (46) for starter preparation and with the yeast separation equipment (54) and feeding pump (55) of slime pulp column (56), which is connected with the container (59) of crude ethanol through the slime pulp column cooler (57) and the crude ethanol cooler (58), the crude ethanol container (59) is interconnected with the waterless ethanol container (61) through the rectification column and through the dewatering device (60).

7. Equipment according to any of the claims 2 - 6, **characterized by** the fact that the conversing and transmitting press (62) comprises four zones,
first zone is made by the feeder (63), which is represented by the cylindric body (68) and cylindric spindle (69) with a constant lead of spindle worm, and cylindric body (68) is provided with the sandwich perforation (71) under the axis of revolution,
second zone is made also by cylindric body and cylindric spindle with constant lead of worm and with the volume contraction of thread profile, and cylindric body is provided with sandwich perforation as well,
third zone is created by conical body (72) and conical spindle (73) with the decreasing lead of helix, and the conical body is furnished with the system of conical areas and small radial channels, and
fourth zone is created by the pressure feeding head (74) of cylindric shape, and the driving gear unit (64) with transmission interconnected with the spindle can be provided by a switching ampermeter (65), eventually interconnected with the fluid feeding equipment (66).

## Patentansprüche

1. Herstellungsmethoden von Monosacchariden, Ethanol, Furfurol, Furan, Methanol, Essigsäure, Aminosäure und Lignin aus recycelten Polymeren mit kontinuierlicher thermischer Druckhydrolyse in Kombination mit Enzymhydrolyse, wo:
- die hydrolysierte Suspension dehnt sich unter mittlerem und normalem atmosphärischen Druck mindestens um zwei Grad aus, wobei sie sich auf Dampfbasis splittet und neben Wasser aus Furfurol, Methanol, Essigsäure, Aminosäure enthält, und auf flüssiger Basis mit einer Wasserlösung von hydrolysen Zuckern und weiteren löslichen Stoffen, sowie in eine feste nicht reagierte Ligno-Zellulosephase,
- die nicht reagierte feste Ligno-Zellulosephase separiert sich unter dem Druck und/oder der Filterung, und nach dem Hinzufügen von Wasser wird mit Zellulosenzymen auf sie eingewirkt, und bei dieser Reaktion entsteht Monosaccharid,
- die Monosaccharide unterziehen sich nach der Separierung des nicht aufgespalteten Lignins einer amylolythischen Hydrolyse, zusammen mit der Einwirkung von Stärkemitteln und der Flüssigphase, wonach die Zuckerflüssigkeit weiterverarbeitet wird, und/oder die Zuckerflüssigkeit als süßer Dampf in Ethanol fermentiert, was nach der Trennung der Gärstoffe entwässert und konzentriert wird,
diese Methode dadurch gegenzeichnet, dass sie die folgenden Elemente enthält:
a) recyclefähiger Rohstoff eines polymären Materials wird in Partikel mit einer Größe von 10 - 30 mm desintegriert, bevor der er der kontinuierlichen zweistufigen thermischen Druckhydrolyse ausgesetzt wird;
b) das desintegrierte Material wird mit dem am Eingang in die Flülldruckpresse einspritze Wasser benetzt, und zwar mit Warmwasser von 20 bis 40°C in einer Menge von 5 bis 10 % Prozent pro Masse des eintretenden Materials,
c) die kontinuierliche thermische Druckhydrolyse wird in einem zweistufigen Prozell durchgeführt, wobei:
- in der ersten Etappe die Temperatur 150 bis 185°C beträgt und der Druck 0,6 bis 1,0 MPa bei einem Hydromodul von 1:4,
- in der zweiten Etappe wird zusätzlich warmes Druckwasser mit einer Temperatur von 200 bis 240°C und einem Druck von 1,6 bis 3,3 MPa eingespritzt,
und dabei wird gleichzeitig die Chlorwasserstoffflüssigkeit oder eine andere geeignete Säure mit einem Volumen von 0,1 bis 1 % der Masse eingespritzt, bezogen auf die Suspension beim Hydromodul 1:3 bis 1:4,
und die Flüssigphase separiert sich nach der Beendigung der Hydrolyse, und nach der Verdickung wird sie als Rohstoff für die Fermentierung in Ethanol oder Furfurol verwendet.

2. Die Anlage zur Durchführung Produktionsmethode gemäß dem Anspruch 1, bestehend aus der Anlage (1) für die Vorbereitung der primären Rohstoffe, des Behälters (2), der Druckfüllstation (4) mit mindestens zwei Hydrolysen, die miteinander verbunden sind, durch den Mitteldruckexpander und die Niederdruckexpander (6) mit dem Schiebe- und Mischbehälter (7) des Hydrolyseproduktes, und das Oberteil des Mitteldruckexpanders und der Niederdruckexpander (6) sind mit dem Oberteil der Rektifikationskolonne (13) des Furfurals und mit dem Furfuralbehälter (14) verbunden, ferner beinhaltet dieses Anlage den Fermentierungsbehälter und die Destillationskolonnen für den fermentierten Ethanoldampf, dadurch gegenzeichnet, dass die zweistufige Hydrolyse zwischen dem ersten und dem zweiten Hydrolyser eine Dekompressions-, Wende- und Übertragungspresse (62) hat, welche die unterschiedlichen Drucke zwischen den Hydrolysern halten kann.

3. Die Anlage gemäß dem Anspruch 2, dadurch gegenzeichnet, dass der Behälter (14) der Fufuralmischung mit der Rektifikationskolonne (13) verbunden ist, die mit dem Dekanter (17) durch das Rohr für Methanol und Wasser verbunden ist, der Dekanter ist mit dem Behälter (18) für den Furfural verbunden ist, der Behälter (19) für den geringprozentigen Furfural mit Wasserist mit der Mentanolkolonne (20) verbunden und diese Kolonne ist an die Behälter (22) und (23) für Methanol verbunden, der untere Teil der Mentanolkolonne (20) ist mit dem Behälter (14) der Furfuralmischung verbunden, der Furfuralbehälter (19) ist mit der Vakuumrektifikationskolonnen (21) verbunden, deren Oberteil an den Dekanter (17) angeschlossen ist, und dessen Unterteil mit dem Behälter (24) des reinen Furfurals (Volumenkonzentration 99,67 %) verbunden ist, die Rektifikationskolonne (21) ist ferner mit dem Behälter (23) für Methanol verbunden.

4. Die Anlage gemäß dem Anspruch 3, dadurch gegenzeichnet, dass der Behälter (24) des reinen Furfurals mit dem Schmelzdruckofen (25) verbunden ist, an den der Behälter (27) des Katalysators angeschlossen ist, und der mit dem Kohlenoxidrohr an den Oxidationsofen (26) angeschlossen ist, das untere Teil des Schmelzdruckofens (25) ist über den Zwischenbehälter (28) mit dem Furanbehälter (29) verbunden.

5. Die Anlage gemäß eines beliebigen Anspruchs 2-3, dadurch gegenzeichnet, dass der Behälter (15) für die Säuren und der Behälter (30) für Etylazethat an die Extrationskolonne (31) angeschlossen ist, die Ableitung aus dieser Extrationskolonne (31) führt in den Behälter (32) für Abwasser, dessen Unterteil mit dem Behälter (39) des Abwassers verbunden ist, dessen Oberteil mit dem Deknater (35) verbunden ist, der mit dem Behälter (30) für das Ethylacetat verbunden ist, die Rektifikationskolonne ist ferner mit dem Behälter (33) für die Säuremischung, das Etylacetat und Wasser verbunden, der mit der Rektifikationskolonne (36) des Ethylacetats verbunden ist, dessen Oberteil mit dem Ethylacetatbehälter (30) verbunden ist, und dessen Unterteil mit den Säurbehälter (37) verbunden ist, der mit dem Behälter (33) für die Säuremischung verbunden ist, und dessen Unterteil mit dem Behälter (40) für reine Säuren verbunden ist.

6. Die Anlage gemäß einem der Ansprüche 2 bis 6, dadurch gegenzeichnet, dass der Behälter (11) für die Zuckerflüssigkeit, sowie der Teil der Thermodruck- und der Enzymhydrolyse direkt an den beheizten Verflüssigungsbehälter (49) angeschlossen ist, und der Warmwasserbehälter (44) und der Behältern (43) für die zermahlene Stärkerohstoff sind an den Apparat (48) für die Zubereitung der Suspension angeschlossen, der an den beheizbaren Verflüssigungsbehälter (49) angeschlossen ist, der mit dem Saccharierungsbehälter (50) verbunden ist, der mit dem Vorbereitungsbehälter (45) für die Zubereitung der Amylolythischen Enzyme und mit der Süßdampfpumpe (51) verbunden ist, diese Pumpe ist mit den Fermentoren (52) und (53) verbunden, die mit dem Behälter (46) für die Zubereitung des Gärstoffs und mit dem Gärstoffseparator (54) und der Dosierungspumpe (55) der Maischebreikolonne (56) verbunden ist, die über den Kühler (57) der Maischebreikolonne (56) und dem Kühler (58) des rohen Alkohols mit dem Behälter (59) des rohen Alkohols verbunden ist, der Behälter (59) für rohen Alkohol ist mit dem Behälter (61) des wasserfreien Alkohols über die Rektifikationskolonne und der Entwässerungseinheit (60) verbunden.

7. Die Anlage gemäß einem der Ansprüche 2 - 6, dadurch gegenzeichnet, dass die Wende- und Transportpresse (62) aus vier Zonen besteht, die erste Zone besteht aus dem Zuteiler (63), der aus einem Walzenkörper (68) und einem Walzbohrer (69) mit konstantem Anstieg des Schraubgewindes besteht, und der Walzenkörper ist unter der Drehachse mit einer Sandwich-Perforierung (71) versehen, die zweite Zone besteht ebenfalls aus einem Walzkörper und einem Walzbohrer mit konstantem Schraubanstieg und mit einem sich verkleinernden Profil des Gewindes, und der Walzenkörer ist ebenfalls mit einer Sandwichperforierung versehen, die dritte Zone besteht aus eine konischen Körper (72) und einem konischen Bohrer (73) mit sinkendem Anstieg des Schraubkopfel, wobei der konische Körper mit Kegelflächen und kleinen radialen Kanälen ausgestattet ist, und die vierte Zone besteht aus der Druckdosierung durch den Kopf (74) in Walzenform, und die Treibstoffeinheit (64) mit der Übertragung, die due der Spindelung angeschlossen ist, kann mit einem Schaltampermeter (65) versehen werden, ggf. mit der Einspritzanlage (66) für Flüssigkeiten.

## Revendications

1. Mode de production de monosaccharides, éthanol, furfural, furane, méthanol, acide acétique, acide formique et lignine issus de matériaux polymères renouvelables par hydrolyse thermique sous pression, combinée à une hydrolyse enzymatique, où :
- la suspension hydrolysée se répand à une pression atmosphérique moyenne et normale au moins en deux niveaux, ce par quoi elle se divise en phase de vapeur comportant, hormis de l'eau, également du furfural, du méthanol, de l'acide acétique, de l'acide formique et en phase liquide comportant une solution aqueuse de sucres d'hydrolyse et d'autres substances dissolubles et en une phase solide lignocellulosique non réactivée,
- la phase solide lignocellulosique non réactivée se sépare par pression et/ou filtration et, après adjonction d'eau, elle y agit par des enzymes cellulolytiques, des monosaccharides naissant lors de cette réaction,
- les monosaccharides sont soumis à une hydrolyse amylolytique après séparation de la lignine non divisée, avec l'action de matières amidonnées et de la phase liquide, après laquelle la solution de sucre continue à être traitée, et/ou après laquelle la solution de sucre en tant que jus sucré fermente en éthanol, qui est desséché et concentré après séparation des levures,
mode de production **se caractérisant par le fait qu'**il comporte les caractéristiques suivante :
a) matière première renouvelable de matériau polymère se désintégrant en des particules de taille 10 - 30 mm avant exposition à une hydrolyse continue thermique sous pression à deux niveaux,
b) matériau désintégré s'humidifiant par de l'eau injectée sur l'entrée dans une presse de remplissage sous pression, par utilisation d'eau chaude d'une température de 20 à 40 °C dans une quantité de 5 à 10 % massiques en poids du matériau d'entrée,
c) l'hydrolyse continue thermique sous pression s'effectuant par un processus à deux
niveaux, où
dans le premier niveau à une température de 150 à 185°C et une pression de 0,6 à 1,0 MPa avec un hydromodule de 1:4,
et dans le deuxième niveau, elle est aspergée de manière complémentaire par de l'eau chaude sous pression d'une température de 200 à 240°C et d'une pression de 1,6 à 3,3 MPa,
de l'acide chlorhydrique ou un autre acide approprié d'un volume de 0,1 à 1 % massique, rapporté à la suspension avec un hydromodule de 1:3 à 1:4, étant injecté dans le même temps, la phase liquide se séparant après achèvement de l'hydrolyse et étant utilisée après son épaississement en tant que matière première pour la fermentation en éthanol ou en furfural.

2. Installation pour la réalisation du mode de production en vertu de la revendication 1, se composant d'une installation (1) pour la préparation des matières premières d'entrée, d'un réservoir (2), d'un dispositif de remplissage sous pression (4), avec au moins deux hydrolyseurs, qui sont raccordés, à travers un expanseur à moyenne pression et un expanseur à basse pression (6), à un réservoir mobile et un réservoir mélangeur (7) de produit d'hydrolyse, la partie supérieure de l'expanseur à moyenne pression et de l'expanseur à basse pression (6) étant raccordée à la partie supérieure d'une colonne de rectification (13) du furfural et à un réservoir de furfural (14), cette installation comportant, en outre, des réservoirs de fermentation et des colonnes de distillation pour le jus fermenté contenant de l'éthanol, **se caractérisant par le fait que** l'hydrolyse à deux niveaux possède entre le premier et le deuxième hydrolyseur une presse de décompression, de séparation et de transfert (62), pouvant maintenir des pressions différentes entre les hydrolyseurs.

3. Installation en vertu de la revendication 2, **se caractérisant par le fait que** le réservoir (14) de mélange de furfural est raccordé à la colonne de rectification (13), qui est raccordée à un décanteur (17) par la conduite pour le méthanol et l'eau, le décanteur étant raccordé à un réservoir (18) pour le furfural à faible pourcentage avec de l'eau et à un réservoir (19) de furfural, le réservoir à furfural à faible pourcentage avec de l'eau étant raccordé à une colonne de méthanol (20), cette colonne étant raccordée aux réservoirs (22) et (23) de méthanol, la partie inférieure de la colonne de méthanol (20) étant raccordée au réservoir (14) de mélange de furfural, le réservoir de furfural (19) étant raccordé à une colonne de rectification sous vide (21), dont la partie supérieure est raccordée au décanteur (17), et dont la partie inférieure est raccordée à un réservoir (24) de furfural pur (concentration volumique 99,67 %), la colonne de rectification (21) étant, en outre, raccordée au réservoir (23) de méthanol.

4. Installation en vertu de la revendication 3, **se caractérisant par le fait que** le réservoir (24) de furfural pur est lié à un four de fusion sous pression (25), auquel est raccordé un réservoir (27) de catalyseur, et qui est raccordé par une conduite pour l'oxyde de carbone à un four d'oxydation (26), la partie inférieure du four de fusion sous pression (25) étant raccordée à travers un réservoir intermédiaire (28) à un réservoir de furane (29).

5. Installation en vertu de l'une des revendications 2-3, **se caractérisant par le fait qu'**un réservoir (15) d'acides et un réservoir (30) d'acétate d'éthyle sont raccordés à une colonne d'extraction (31), l'évacuation de cette colonne d'extraction (31) étant menée dans un réservoir (32) pour l'eau de rebut, dont la partie inférieure est raccordée à un réservoir (39) d'eau de rebut, dont la partie supérieure est raccordée à un décanteur (35), qui est raccordé au réservoir (30) d'acétate d'éthyle, une colonne de rectification étant, en outre, raccordée à un réservoir (33) pour le mélange d'acides, d'acétate d'éthyle et d'eau, qui est raccordé à une colonne de rectification (36) d'acétate d'éthyle, dont la partie supérieure est raccordée au réservoir (30) d'acétate d'éthyle, et dont la partie inférieure est raccordée à un réservoir (37) d'acides, qui est raccordé au réservoir (33) pour le mélange d'acides, et dont la partie inférieure est raccordée à un réservoir (40) d'acides purs.

6. Installation en vertu de l'une des revendications 2 à 6, **se caractérisant par le fait qu'**un réservoir (11) de solution de sucre, en tant que partie de l'hydrolyse thermique sous pression et enzymatique, est directement raccordé à un réservoir chauffé de liquéfaction (49), un réservoir (44) d'eau chaude et un réservoir (43) de matière première amidonnée broyée étant raccordés à un appareil (48) pour la préparation d'une suspension, qui est raccordé au réservoir chauffé de liquéfaction (49), qui est raccordé à un réservoir de saccharification (50), qui est raccordé à un réservoir de préparation (45) pour la préparation d'enzymes amylolytiques et à une pompe (51) de jus sucré, cette pompe étant raccordée à des fermenteurs (52) et (53), qui sont raccordés à un récipient (46) pour la préparation de ferment et à un séparateur de levures (54) et la pompe de dosage (55) d'une colonne de moût (56), qui est raccordée à travers le refroidisseur (57) de la colonne de moût (56) et un refroidisseur (58) d'alcool brut à un réservoir (59) d'alcool brut, le réservoir (59) d'alcool brut étant raccordé à un réservoir (61) d'alcool absolu à travers une colonne de rectification et une unité d'évacuation (60).

7. Installation en vertu de l'une des revendications 2 - 6, **se caractérisant par le fait que** la presse de séparation et de transfert (62) est composée de quatre zones, la première zone étant formée d'un dispositif d'alimentation (63), composé d'un corps cylindrique (68) et d'une broche cylindrique (69) avec montée constante de l'hélice de la broche, le corps cylindrique étant muni sous l'axe de rotation d'une perforation de sandwich (71), la deuxième zone étant, de même, formée d'un corps cylindrique et d'une broche cylindrique avec montée constante de l'hélice et volume diminuant de profil du filetage, le corps cylindrique étant, de même, muni d'une perforation de sandwich, la troisième zone étant formée d'un corps conique (72) et d'une broche conique (73) avec montée descendante de l'hélice, le corps conique étant équipé d'un système de surfaces coniques et de petits canaux radiaux, la quatrième zone étant formée d'une tête de dosage sous pression (74) de forme cylindrique, l'unité de commande (64) avec transmission, raccordée à la broche, pouvant être munie d'un ampèremètre de commutation (65), éventuellement raccordé à un dispositif d'injection (66) de liquide.
